# FASCICULE DE BREVET EUROPEEN

(11) **EP 3 796 865 B1**
(45) Date de publication et mention de la délivrance du brevet: **18.12.2024**
(21) Numéro de dépôt: 19725305.7
(22) Date de dépôt: 14.05.2019
(51) Int. Cl.: A61C 7/00, A61C 9/00, G06T 7/00

(54) **DISPOSITIF D'ANALYSE D'UNE SITUATION DENTAIRE**
VORRICHTUNG ZUR ANALYSE EINER ZAHNSITUATION
DEVICE FOR ANALYSING A DENTAL SITUATION

(30) Priorité: 22.05.2018 EP 18305627
(43) Date de publication de la demande: 31.03.2021
(73) Titulaire: Dental Monitoring, 75017 Paris (FR)
(72) Inventeur: SALAH, Philippe, 75020 PARIS (FR); PELLISSARD, Thomas, 92110 CLICHY (FR); GHYSELINCK, Guillaume, 59169 CANTIN (FR); DEBRAUX, Laurent, 75020 PARIS (FR)
(74) Mandataire: Novagraaf Group
(86) Numéro de dépôt international: PCT/EP2019/062380
(87) Numéro de publication internationale: WO 2019/224055

(56) Documents cités:
- EP-A1- 3 050 512
- WO-A1-2017/182648
- FR-A1- 3 032 282
- US-A1- 2004 197 727
- US-A1- 2015 182 296
- US-A1- 2017 065 379
- US-A1- 2017 281 313
- US-A1- 2018 055 600
- US-A1- 2018 110 590
- US-A1- 2018 125 610

## Description

### Domaine technique

La présente invention concerne l'analyse d'une situation dentaire d'un patient.

### Etat de la technique

Lorsqu'un professionnel des soins dentaires intervient sur les dents d'un patient ou qu'il effectue un diagnostic, il a besoin d'évaluer la situation dentaire réelle, et en particulier le bon positionnement d'un appareil orthodontique fixé sur les dents.

Cette évaluation requiert généralement une comparaison de la situation dentaire réelle avec une situation dentaire théorique. Par exemple, pour diagnostiquer le détachement d'une attache d'un appareil orthodontique, il peut être nécessaire de comparer la situation dentaire réelle dans laquelle l'attache est écartée de manière anormale de la dent sur laquelle elle est supposée être collée, avec une situation dentaire théorique dans laquelle ladite attache est effectivement collée sur ladite dent. Ce besoin existe notamment lors de traitements sur mesure, pour lesquels les attaches sont conformées pour s'adapter précisément à la morphologie des dents et/ou selon les prescriptions spécifiques du professionnel des soins dentaires.

De manière générale, la qualité de la comparaison entre la situation dentaire réelle et la situation dentaire théorique a un impact sur la qualité du traitement, qu'il soit thérapeutique ou non.

Il existe donc un besoin permanent pour un dispositif d'analyse permettant d'améliorer l'appréciation d'une situation dentaire réelle, notamment par un professionnel des soins dentaires.

Par ailleurs, l'efficacité d'un traitement dentaire est directement liée au respect de la prescription médicale par le patient. A défaut d'une bonne observance, la situation dentaire peut empirer, ce qui entraîne un risque supplémentaire pour le patient, mais également des coûts pour la Sécurité Sociale ou les organismes de financement des soins.

La mauvaise observance d'un traitement rend également plus difficiles les études cliniques, en particulier si le patient ne déclare pas les périodes de non observance.

Le document US2018110590 décrit un dispositif de réalité virtuelle permettant de prévisualiser la position et l'esthétique d'un appareil orthodontique par superposition d'un modèle de traitement dentaire virtuel aligné sur l'arcade physique du patient.

Il existe donc un besoin pour un dispositif permettant d'améliorer l'observance.

Un but de l'invention est de répondre, au moins partiellement, à ces besoins.

### Résumé de l'invention

L'invention propose un dispositif pour la mise en oeuvre d'un procédé d'analyse d'une situation dentaire réelle d'un patient tel que dans la revendication 1.

Dans un mode de réalisation particulier, l'étape B) consiste en une simulation, pour un instant de simulation, de la scène dentaire réelle représentée sur l'image actualisée, puis en une détermination d'une scène dentaire virtuelle en fonction de ladite simulation.

Comme on le verra plus en détail dans la suite de la description, le dispositif selon l'invention permet donc à l'opérateur d'observer simultanément
- la scène dentaire réelle, directement ou à travers un écran d'une part, ou sur l'image actualisée d'autre part, et
- la scène dentaire virtuelle, en superposition avec la scène dentaire réelle ou avec la représentation de la scène dentaire réelle sur l'image actualisée, respectivement.

Cette superposition facilite considérablement l'analyse, par l'opérateur, de préférence en temps réel, des différences entre la scène dentaire réelle et la scène dentaire virtuelle. Elle permet également d'apporter des informations relatives à la scène dentaire réelle et de les disposer en fonction de la représentation de la scène dentaire réelle sur l'image actualisée. La qualité de la transmission de ces informations à l'opérateur en est améliorée. Enfin, elle permet de simuler de manière réaliste une situation dentaire, par exemple de simuler un traitement esthétique.

Le procédé mis en oeuvre par le dispositif selon l'invention, ou « procédé d'analyse », peut encore comporter une ou plusieurs des caractéristiques optionnelles suivantes :
- à l'étape B), la scène dentaire virtuelle comporte
   - la représentation d'un ou plusieurs éléments physiques de la scène dentaire réelle modélisés par le modèle de référence, et/ou
   - la représentation d'un ou plusieurs éléments physiques de la scène dentaire réelle non modélisés par le modèle de référence, et/ou
   - la représentation d'un ou plusieurs éléments physiques qui ne sont pas dans la scène dentaire réelle et/ou qui ne sont pas dans le modèle de référence, et/ou
   - un indicateur qui ne représente pas un élément physique de la scène dentaire réelle, de préférence plusieurs tels indicateurs ;
- le critère d'évaluation est relatif à l'état de santé du patient et/ou à un coût pour un traitement dentaire ;
- le message est une consigne à suivre par le patient et/ou par un professionnel des soins dentaires en charge du patient ;
- à l'étape B), on détermine la nature et/ou l'emplacement et/ou l'apparence d'un élément de la scène dentaire virtuelle, et notamment d'un indicateur, en fonction du contexte, et en particulier en fonction de la distance à un autre élément de la scène dentaire virtuelle et/ou en fonction d'un objectif poursuivi ;
- à l'étape B), la nature et/ou **l'emplacement** et/ou **l'apparence** d'éléments de la scène dentaire virtuelle sont déterminés en fonction de la nature et/ou de l'emplacement et/ou de l'apparence d'éléments affichés dans des scènes dentaires virtuelles antérieures, c'est-à-dire de cycles d'étapes A) à C) antérieurs, et/ou en fonction de l'intervalle temporel avec lesdites scènes dentaires virtuelles antérieures, et/ou en fonction de différences entre les images actualisées de cycles successifs des étapes A) à C) ;
- à l'étape B), on détermine la scène dentaire virtuelle de manière qu'elle comporte des informations, notamment textuelles ou graphiques, sur
   - des différences entre l'image actualisée et l'image de référence, et/ou
   - des différences entre le modèle de référence et la scène dentaire réelle ;
- à l'étape C), on présente la scène dentaire virtuelle sur la scène dentaire réelle ou sur l'image actualisée de manière que les représentations d'éléments physiques réels de la scène dentaire virtuelle se superposent, de manière réaliste, avec lesdits éléments physiques réels ou avec les représentations desdits éléments physiques réels sur l'image actualisée, respectivement.

Selon un aspect de l'invention, à l'étape C), la scène dentaire virtuelle est présentée sur un écran transparent, en superposition avec la scène dentaire réelle qu'un opérateur peut observer à travers l'écran, en particulier lorsqu'il est face à l'écran.

De préférence, l'écran et la caméra sont immobilisés par rapport à l'opérateur. De préférence, ils sont intégrés dans des lunettes. Le procédé selon l'invention est alors particulièrement utile pour qu'un professionnel des soins dentaires portant les lunettes puisse évaluer, en temps réel, la situation dentaire d'un patient qu'il examine.

Selon un aspect de l'invention, à l'étape C), la scène dentaire virtuelle est présentée, en superposition avec la représentation de la scène dentaire réelle sur l'image actualisée, sur un écran opaque, c'est-à-dire à travers lequel l'opérateur ne peut pas voir.

Dans un mode de réalisation, l'écran est celui d'un téléphone portable, d'une tablette, d'un ordinateur portable, ou d'un casque de réalité virtuelle. L'écran peut être également la glace d'un miroir, de préférence équipé d'au moins une caméra.

De préférence, l'opérateur manipule un téléphone portable, un ordinateur portable, un casque de réalité virtuelle ou un miroir équipé d'au moins une caméra, pour acquérir ladite image actualisée et visualiser la scène dentaire réelle et la scène dentaire virtuelle.

Dans un mode de réalisation, à l'étape B) ou d), la scène dentaire virtuelle est déterminée en fonction d'une valeur d'au moins un paramètre de traitement modifiable par interaction avec ledit téléphone portable, ledit ordinateur portable, ledit casque de réalité virtuelle, ledit appareil photo ou ledit miroir ou lesdites lunettes, de préférence avec le téléphone portable. De préférence, le paramètre de traitement est relatif à un port d'un appareil orthodontique par le patient et/ou au respect d'une consigne de traitement par le patient.

Le procédé d'analyse peut faciliter les décisions du patient, en particulier parce qu'il lui permet, en choisissant l'instant de simulation, de visualiser l'effet, sur son apparence, des différentes options de traitement possibles. Le procédé d' analyse peut être également un outil pédagogique efficace pour améliorer l'observance.

### Utilisation d'un modèle de référence

Dans un mode de réalisation préféré, on détermine la scène dentaire virtuelle au moyen d'un modèle numérique tridimensionnel modélisant numériquement au moins une arcade du patient, appelé « modèle de référence ».

De préférence, le modèle de référence sert également, à l'étape C), pour positionner la scène dentaire virtuelle par rapport à la scène dentaire réelle ou à sa représentation.

Avantageusement, la composition de la scène dentaire virtuelle peut être ainsi entièrement automatisée.

Le procédé d'analyse peut en particulier comporter les étapes successives suivantes :
a) à un instant de référence, génération, de préférence au moyen d'un scanner, d'un modèle de référence modélisant numériquement au moins une arcade dentaire du patient;
b) optionnellement, modification du modèle de référence ;
c) à un instant actualisé, acquisition, de préférence au moyen d'une caméra, d'une image actualisée représentant une scène dentaire réelle;
d) recherche, par observation du modèle de référence, optionnellement modifié, d'une image de référence présentant une concordance maximale avec l'image actualisée, puis détermination, en fonction de l'image de référence, d'une scène dentaire virtuelle ;
e) présentation de la scène dentaire virtuelle
   - en transparence et en superposition sur la scène dentaire réelle, en particulier sur un écran à travers lequel la scène dentaire réelle est visible ou en projection sur la scène dentaire réelle, ou
   - en superposition avec la représentation de la scène dentaire réelle sur l'image actualisée affichée sur un écran, en transparence ou non sur ladite représentation,
puis, de préférence, reprise à l'étape c).

L'étape c) est un cas particulier d'étape A). Les caractéristiques optionnelles applicables à l'étape A) sont donc applicables à l'étape c).

Les étapes a), b) et d) constituent ensemble un cas particulier d'étape B). Les caractéristiques optionnelles applicables à l'étape B) sont donc applicables aux étapes a), b) et d).

L'étape e) est un cas particulier d'étape C). Les caractéristiques optionnelles applicables à l'étape C) sont donc applicables à l'étape e).

De préférence, à l'étape a), on scanne une arcade du patient ou un modèle physique de ladite arcade alors que ladite arcade ou ledit modèle physique de ladite arcade porte ou ne porte pas un appareil orthodontique, et en particulier alors que ladite arcade ou ledit modèle physique de ladite arcade ne porte pas un appareil orthodontique.

L'instant actualisé peut être différent de l'instant de référence, et en particulier être postérieur de plus de 3 jours à l'instant de référence. Le procédé d'analyse peut alors être utilisé pour visualiser les modifications de l'arcade entre ces deux instants.

Dans un mode de réalisation, la scène dentaire virtuelle est constituée par l'image de référence.

De préférence, à l'étape d), on détermine la nature et/ou l'emplacement et/ou l'apparence d'un élément de la scène dentaire virtuelle, et notamment d'un indicateur, en fonction :
- de l'image de référence, et/ou
- de différences entre l'image actualisée et l'image de référence, et/ou
- du contexte.

Le modèle de référence peut donner accès à des informations « cachées », c'est-à-dire qui ne sont pas accessibles ou qui sont difficilement accessibles à l'opérateur. Ces informations cachées peuvent en particulier dépendent de l'image de référence, et donc de l'image actualisée. Dans un mode de réalisation, au moins une partie des informations cachées sont sélectionnées à l'étape d). L'étape d) permet ainsi de créer une scène dentaire virtuelle qui contient de telles informations et l'étape e) permet de les présenter sur la scène dentaire réelle ou sur sa représentation.

### Applications

Le procédé d'analyse peut être en particulier utilisé à des fins non thérapeutiques, en particulier à des fins de recherche, par exemple pour évaluer l'efficacité d'un traitement ou d'un appareil orthodontique, ou à des fins esthétiques, ou à des fins pédagogiques.

Dans un mode de réalisation, la scène dentaire virtuelle peut être relative à la scène dentaire réelle telle que simulée à un instant de simulation ou, pour le passé, telle qu'observée à un instant de simulation. En particulier, la scène dentaire virtuelle peut simuler la scène dentaire réelle à l'instant de simulation. La simulation est de préférence obtenue au moyen d'un ordinateur, de préférence au moyen d'un algorithme d'intelligence artificielle.

Le procédé d'analyse est alors particulièrement utile pour qu'un opérateur, et en particulier un patient, puisse visualiser une situation dentaire à un instant de simulation antérieur ou, de préférence, postérieur à l'instant actualisé, ou visualiser l'évolution d'une telle situation dentaire en faisant varier l'instant de simulation.

Le procédé d'analyse comprend une série de cycles d'étapes A) à C), ou c) à e), la scène dentaire virtuelle étant déterminée, à chaque cycle, pour simuler une situation dentaire à l'instant de simulation en cours.

Dans un mode de réalisation, on détermine la scène dentaire virtuelle au moyen d'un outil de simulation dynamique configuré pour fournir au moins un élément de la scène dentaire virtuelle en fonction d'un instant de simulation déterminé, en particulier en fonction d'un instant de simulation postérieur à l'instant actualisé.

Lorsqu'il est utilisé à des fins de simulation, le procédé d'analyse peut comporter une ou plusieurs des caractéristiques optionnelles suivantes :
- l'instant de simulation est postérieur à l'instant actualisé, par exemple postérieur de plus de 1 jour, 10 jours ou 100 jours à l'instant actualisé ;
- l'instant de simulation est déterminé avant l'étape A) ou avant l'étape B), de préférence par interaction de l'opérateur avec un écran ;
- ledit écran
   - est un écran tactile et on modifie l'intervalle temporel entre l'instant actualisé et l'instant de simulation par interaction, de préférence par glissement, d'un doigt sur ledit écran, et/ou
   - comporte un champ de saisie de l'instant de simulation ;
- entre deux cycles d'étapes A) à C), on modifie l'intervalle temporel entre l'instant actualisé et l'instant de simulation ;
- on règle l'instant de simulation en modifiant la position d'un curseur représenté sur l'écran ;
- à l'étape B), on détermine la nature et/ou l'emplacement et/ou l'apparence d'un élément de la scène dentaire virtuelle, et notamment d'un indicateur, en fonction :
   - du contexte, et en particulier en fonction de la distance à un autre élément de la scène dentaire virtuelle et/ou en fonction d'un objectif poursuivi, et/ou en fonction de l'instant de simulation ;
- à l'étape B), on détermine, pour l'instant de simulation, une couleur et/ou une forme et/ou une position d'une partie visible ou invisible de la bouche, de préférence d'une mâchoire, d'une couronne dentaire et/ou d'une racine dentaire et/ou de la gencive du patient, et on détermine la scène dentaire virtuelle de manière qu'elle reproduise ladite couleur et/ou forme et/ou position ;
- le patient acquiert ladite image actualisée à l'étape A), et observe l'écran à l'étape C).

Lorsque le procédé comporte les étapes a) à e), à l'étape b), le modèle de référence peut être modifié pour simuler l'effet de l'écoulement du temps entre l'instant de référence et l'instant de simulation, et en particulier l'effet d'un traitement dentaire, thérapeutique ou non, entre ces instants.

Le procédé d'analyse peut être encore notamment utilisé pour :
- évaluer une différence relative à la position et/ou la forme et/ou une dimension d'une ou plusieurs dents et/ou d'un appareil orthodontique entre l'instant de référence et l'instant actualisé, ou entre l'instant de simulation et l'instant actualisé, notamment dans le cadre d'un traitement orthodontique, et/ou
- indiquer un emplacement cible de l'arcade dentaire, en particulier sur une ou plusieurs dents.

Dans un mode de réalisation, la scène dentaire virtuelle est relative à la scène dentaire réelle, de préférence représente au moins en partie la scène dentaire réelle telle qu'elle se présente à l'instant actualisé ou telle qu'elle devrait se présenter à l'instant actualisé d'après une simulation. Autrement dit, si la scène dentaire virtuelle représente les dents du patient, elle les représente dans leur position à l'instant actualisé ou dans leur position simulée à l'instant actualisé. L'instant de simulation est donc identique ou proche de l'instant actualisé, par exemple écarté de l'instant actualisé de moins de 1 mois, moins de 2 semaines ou moins d'une semaine.

### Dispositif

L'invention concerne un dispositif pour la mise en oeuvre du procédé d'analyse.

Un tel dispositif comporte
- optionnellement, des moyens pour générer et optionnellement modifier un modèle de référence aux étapes a) et b), ce moyens comportant de préférence un scanner et un ordinateur ;
- une caméra configurée pour mettre en oeuvre l'étape A) ou c) ;
- une unité de traitement configurée pour mettre en oeuvre l'étape B) ou l'étape d) ;
- des moyens de présentation, sous la forme d'un écran, configurés pour mettre en oeuvre l'étape C) ou e) ;

l'unité de traitement, la caméra et les moyens de présentation étant configurés pour communiquer entre eux pour mettre en oeuvre lesdites étapes.

De préférence, la caméra est configurée de manière à acquérir une succession d'images actualisées de la scène dentaire réelle et transmettre lesdites images actualisées à l'unité de traitement.

De préférence,
- l'unité de traitement est configurée pour :
   - rechercher, pour chaque image actualisée reçue de la caméra, par observation d'un modèle de référence, une image de référence présentant une concordance maximale avec ladite image actualisée,
   - déterminer, en fonction de l'image de référence, une scène dentaire virtuelle, et
   - transmettre ladite scène dentaire virtuelle aux moyens de présentation ;
- les moyens de présentation sont configurés pour faire apparaître la scène dentaire virtuelle en superposition sur la scène dentaire réelle ou sur la représentation de la scène dentaire réelle dans l'image actualisée.

Dans un mode de réalisation, la scène dentaire virtuelle est présentée en transparence sur la scène dentaire réelle ou sur sa représentation dans l'image actualisée.

Dans un mode de réalisation, le dispositif comporte des lunettes dans lesquelles la caméra et les moyens de présentation, de préférence la caméra, les moyens de présentation et l'unité de traitement sont intégrés.

Dans un mode de réalisation, les moyens de présentation sont configurés pour présenter la scène dentaire virtuelle sur un verre des lunettes, en superposition réaliste avec la scène dentaire réelle ou pour projeter la scène dentaire virtuelle sur les éléments physiques qui constituent la scène réelle.

Dans un mode de réalisation, le dispositif comporte un téléphone portable ou un appareil photo ou un ordinateur portable ou un casque de réalité virtuelle ou une tablette ou un miroir, dans lequel la caméra et les moyens de présentation, de préférence la caméra, les moyens de présentation et l'unité de traitement sont intégrés.

Les moyens de présentation comportent de préférence un écran permettant d'afficher l'image actualisée et de présenter la scène dentaire virtuelle sur la représentation de la scène dentaire réelle, en « réalité augmentée ».

La représentation de la scène dentaire réelle peut être une image affichée sur l'écran du téléphone portable ou de l'appareil photo ou de l'ordinateur portable ou du casque de réalité virtuelle ou de la tablette.

La représentation de la scène dentaire réelle peut être le reflet renvoyé par le miroir.

Comme on le verra plus en détail dans la suite de la description, l'observateur peut ainsi observer la scène dentaire réelle ou sa représentation sur l'image actualisée et la scène dentaire virtuelle, de préférence en transparence, sur la scène dentaire réelle ou sur sa représentation sur l'image actualisée. Il peut ainsi immédiatement avoir accès aux informations contenues dans la scène dentaire virtuelle, présentées dans l'environnement de la scène dentaire réelle. L'intégration de l'écran dans des lunettes est particulièrement avantageuse à cet effet.

### Définitions

Un « patient » est une personne pour laquelle le procédé d'analyse est mis en oeuvre, indépendamment du fait que cette personne suive un traitement dentaire ou non.

Une « situation dentaire » définit un ensemble de caractéristiques relatives à une arcade d'un patient à un instant, par exemple la position des dents, leur forme, la position d'un appareil orthodontique, etc. à cet instant.

Par « professionnel des soins dentaires », on entend toute personne qualifiée pour prodiguer des soins dentaires, ce qui inclut un orthodontiste et un dentiste. L'opérateur qui porte les lunettes est de préférence un orthodontiste ou un dentiste.

Par « opérateur », on entend la personne qui met en oeuvre le procédé pour que lui soit présentée la scène dentaire virtuelle à l'étape C). L'opérateur peut être en particulier le patient ou le professionnel de soins dentaires.

Par « lunettes », on entend un appareil qui peut être porté devant les yeux d'un opérateur, de préférence en prenant appui sur le nez et/ou les oreilles de l'opérateur.

Un « appareil orthodontique » est un appareil porté ou destiné à être porté par un patient. Un appareil orthodontique peut être destiné à un traitement thérapeutique ou prophylactique, mais également à un traitement esthétique. Un appareil orthodontique peut être en particulier un appareil à arc et attaches, ou une gouttière orthodontique. Une telle gouttière s'étend de manière à suivre les dents successives de l'arcade sur laquelle elle est fixée. Elle définit une goulotte de forme générale en « U ». La configuration d'un appareil orthodontique peut être en particulier déterminée pour assurer sa fixation sur les dents, mais également en fonction d'un positionnement cible souhaité pour les dents. Plus précisément, la forme est déterminée de manière que, dans la position de service, l'appareil orthodontique exerce des contraintes tendant à déplacer les dents traitées vers leur positionnement cible (appareil orthodontique actif), ou à maintenir les dents dans ce positionnement cible (appareil orthodontique passif, ou « de contention »).

Par « modèle », on entend un modèle tridimensionnel numérique.

Par "image", on entend une image en deux dimensions. Une image est formée de pixels. Les « images actualisées » sont des images prises à un instant dit « actualisé ». Elles sont de préférence extraites d'un film pris par une caméra. Une « image de référence » est une vue d'un modèle « de référence » qui présente une concordance maximale avec une image actualisée.

Par « arcade », on entend tout ou partie d'une arcade dentaire. Par « image d'une arcade », ou « modèle d'une arcade », on entend ainsi une représentation en 2 ou 3 dimensions, respectivement, de tout ou partie de ladite arcade.

Une « scène » est constituée par un ensemble d'éléments qui peuvent être observés simultanément. Une « scène dentaire » est une scène comportant une arcade.

Une « scène réelle » est constituée d'éléments physiques. Une scène dentaire réelle comporte donc une arcade, mais aussi, un appareil orthodontique porté par les dents de l'arcade.

Une « scène virtuelle » est constituée de représentations d'éléments, notamment des représentations
- d'un élément physique de la scène dentaire réelle correspondante (c'est-à-dire de la scène dentaire réelle représentée sur l'image actualisée à partir de laquelle la scène dentaire virtuelle a été déterminée) ou
- d'une autre information, ou « indicateur ».

Un élément physique peut être modélisé par le modèle de référence, par exemple un contour de dent, ou non modélisé par le modèle de référence.

Une représentation d'un élément physique n'est pas nécessairement réaliste.

La présentation d'une scène dentaire virtuelle est « en transparence » sur une scène dentaire réelle lorsqu'elle permet à l'opérateur d'apercevoir, à travers ladite scène dentaire virtuelle, la scène dentaire réelle.

La présentation d'une scène dentaire virtuelle est « en transparence » sur une image actualisée représentant une scène dentaire réelle lorsqu'elle permet à l'opérateur d'apercevoir l'image actualisée à travers ladite scène dentaire virtuelle.

Une scène dentaire virtuelle est affichée « en superposition » ou « en registre » avec une scène dentaire réelle ou avec une représentation d'une scène dentaire réelle sur une image actualisée lorsqu'elle comporte des représentations d'éléments physiques de la scène dentaire réelle qui sont présentées de manière que les contours desdites représentations, éventuellement transparents, se superposent aux contours desdits éléments physiques ou desdites représentations desdits éléments physiques sur l'image actualisée, respectivement. La superposition apparaît ainsi réaliste. Par exemple, lorsque l'observateur observe une dent d'une arcade dentaire, la présentation est déterminée de manière que la représentation de la dent sur la scène dentaire virtuelle vienne se superposer à la vue de cette dent perçue par l'opérateur.

« L'emplacement » d'un élément de la scène dentaire virtuelle fait référence à sa position par rapport aux éléments de la scène dentaire réelle ou de la représentation de ces éléments sur l'image actualisée lorsque la scène dentaire virtuelle est présentée à l'étape C).

On appelle « concordance » (« *match »* ou « fit *» en* anglais) entre deux objets une mesure de la différence, ou « distance », entre ces deux objets.

De préférence, deux images ou « vues » qui présentent une concordance maximale représentent sensiblement les mêmes éléments de la même façon. Autrement dit, les représentations des éléments sur ces deux images sont sensiblement superposables.

Il faut interpréter "comprendre " ou "comporter " ou "présenter " ou « représenter » de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente, schématiquement, les différentes étapes d'un procédé d'analyse selon un mode de réalisation;
- la figure 2 représente un exemple d'une image de référence d'un modèle de référence ;
- les figures 3 (3a-3b) et 4 (4a et 4b) illustrent le traitement d'une image de référence et d'une image actualisée, respectivement, pour en extraire une carte de référence et une carte actualisée, respectivement, représentant une information de contour ;
- les figures 5, 6 et 7 illustrent schématiquement des exemples de dispositifs selon l'invention ;
- la figure 8 représente la superposition d'une scène dentaire virtuelle sur une image actualisée.

Des références identiques sont utilisées pour désigner des organes identiques ou analogues sur les différentes figures.

### Description détaillée

### Dispositif

Un dispositif 10 selon l'invention comporte une caméra 16, une unité de traitement informatique 12 et des moyens de présentation 18. La caméra 16, l'unité de traitement 12 et les moyens de présentation sont pourvus de moyens leur permettant de communiquer entre eux.

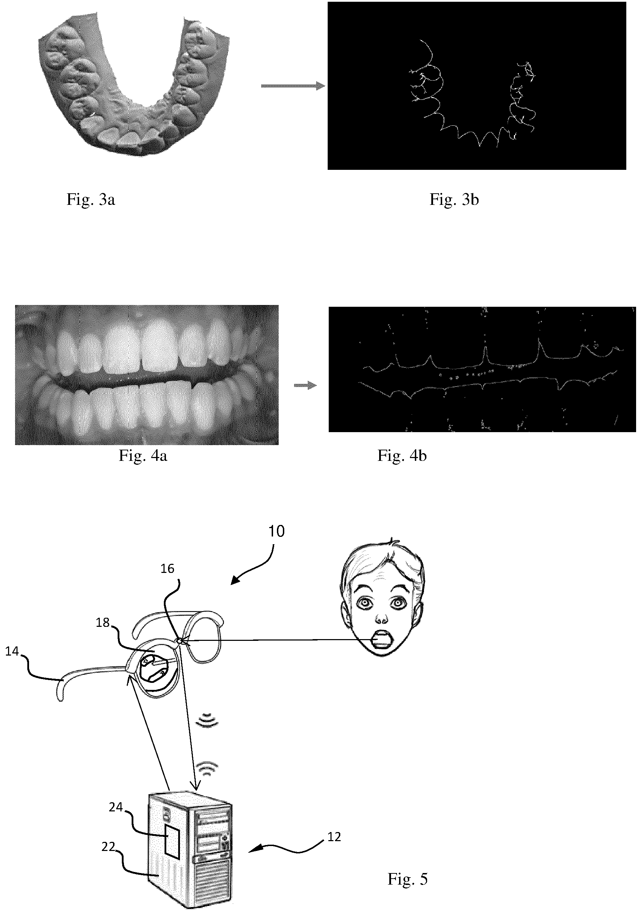

Comme représenté sur les figures 5, 6 et 7, la caméra 16 et/ou l'unité de traitement 12 et/ou les moyens de présentation peuvent être intégrés dans des lunettes comportant classiquement une monture 14 configurée pour être portée par le nez et les oreilles d'un opérateur, dans un téléphone portable, dans une tablette, dans un ordinateur portable, dans un casque de réalité virtuelle, dans un appareil photo ou dans un miroir 17.

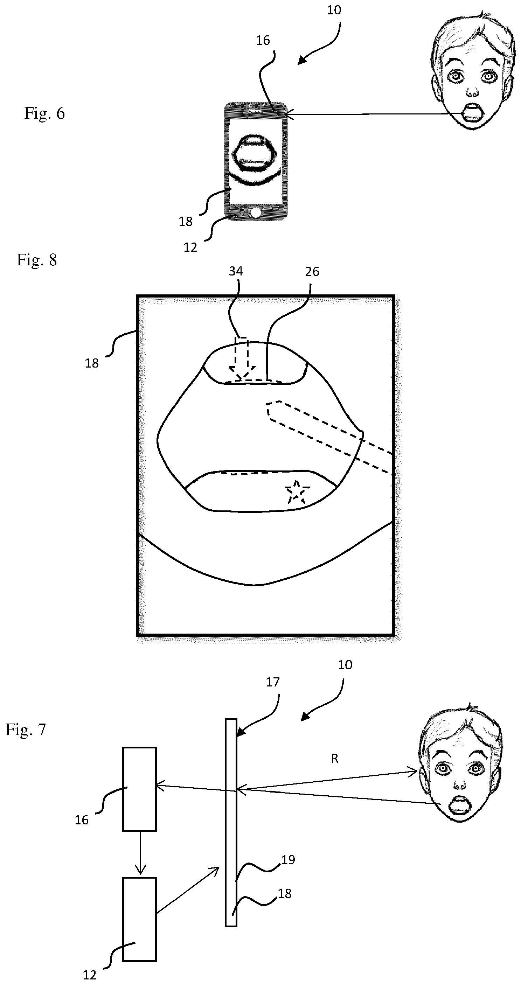

La caméra 16 est destinée à l'acquisition d'images actualisées représentant la scène dentaire réelle observée par l'opérateur à travers les lunettes (figure 5) ou sur l'écran d'un téléphone portable (figure 6) ou d'un ordinateur portable, d'un casque de réalité virtuelle, d'un appareil photo ou d'une tablette, ou sur la glace 19 du miroir (figure 7). Elle peut être une caméra classique.

Dans un mode de réalisation, le dispositif comporte des moyens de projection de motifs de lumière structurée, par exemple d'un nuage de points, par exemple laser, en particulier infrarouge. Avantageusement, la scène dentaire réelle fait apparaître ces motifs de manière déformée, ce qui permet d'en déduire des informations relatives à la profondeur.

Dans le cas où le dispositif comporte un miroir, la glace 19 peut être une glace sans tain et la caméra peut être disposée derrière la glace, comme sur la figure 7.

Dans un mode de réalisation, le dispositif comporte plusieurs caméras, ce qui permet, par simple calcul trigonométrique, d'évaluer la distance entre les objets observés et la caméra. La multiplicité des caméras permet ainsi d'accélérer la recherche des images de référence.

Les caméras permettent ainsi également de simuler la vue tridimensionnelle qu'a l'observateur de la scène dentaire réelle.

Par « moyens de présentation », on entend tout moyen configuré pour recevoir, de l'unité de traitement 12, une scène dentaire virtuelle et faire apparaître ladite scène dentaire virtuelle sur la scène dentaire réelle, sur un écran à travers lequel un observateur peut voir la scène dentaire réelle ou sur l'image actualisée.

Dans un mode de réalisation, les moyens de présentation 18 comportent un écran et un projecteur pour projeter sur l'écran la scène dentaire virtuelle.

En particulier dans le mode de réalisation dans lequel la caméra est intégrée dans des lunettes, l'écran peut être transparent. Il est de préférence fixé sur la monture 14, et la scène dentaire virtuelle est présentée sur l'écran afin que l'opérateur voie, en observant cet écran, à la fois la scène dentaire réelle à travers l'écran, mais aussi la scène dentaire virtuelle projetée sur l'écran.

Les lunettes comportent classiquement deux verres qui s'étendent devant les deux yeux de l'opérateur, respectivement, mais le nombre de verres n'est pas limitatif. Les verres peuvent être correcteurs de la vision ou non. Plusieurs verres peuvent s'étendre devant un même oeil. Par exemple, un premier verre peut corriger la vue de l'opérateur et un deuxième verre peut être un verre servant d'écran.

L'appareil HoloLens, développé par la société Microsoft^{®}, est un exemple de moyens de présentation.

L'écran peut être opaque et l'image actualisée et la scène dentaire virtuelle sont présentées sur l'écran, en registre, afin que l'opérateur voie, en observant cet écran, à la fois l'image actualisée représentant la scène dentaire réelle, mais aussi la scène dentaire virtuelle.

Dans ce mode de réalisation notamment, la caméra peut être immobilisée par rapport à l'écran, par exemple quand la caméra et l'écran sont intégrés dans un téléphone portable (figure 6) ou un ordinateur portable ou un casque de réalité virtuelle ou un appareil photo ou une tablette ou un miroir

Alternativement, la caméra peut être libre (ou « indépendante ») par rapport à l'écran. Avantageusement, ce dernier mode de réalisation permet une grande souplesse. Par exemple, dans un mode de réalisation, la caméra peut être introduite à l'intérieur de la bouche alors que l'écran reste à l'extérieur de la bouche.

En particulier dans le mode de réalisation dans lequel la caméra est intégrée dans un miroir ou est solidaire d'un miroir, l'écran peut être la glace 19 réfléchissante du miroir, et la scène dentaire virtuelle peut être présentée sur l'écran, en registre avec le reflet R renvoyé par la glace 19, afin que l'opérateur voie, en observant cet écran, à la fois le reflet R représentant la scène dentaire réelle, mais aussi la scène dentaire virtuelle superposée à la scène dentaire réelle.

Le reflet, comme l'image actualisée affichée sur l'écran des lunettes, du téléphone portable, de l'ordinateur portable, du casque de réalité virtuelle, de l'appareil photo ou de la tablette, donne ainsi à l'opérateur une représentation de scène dentaire réelle.

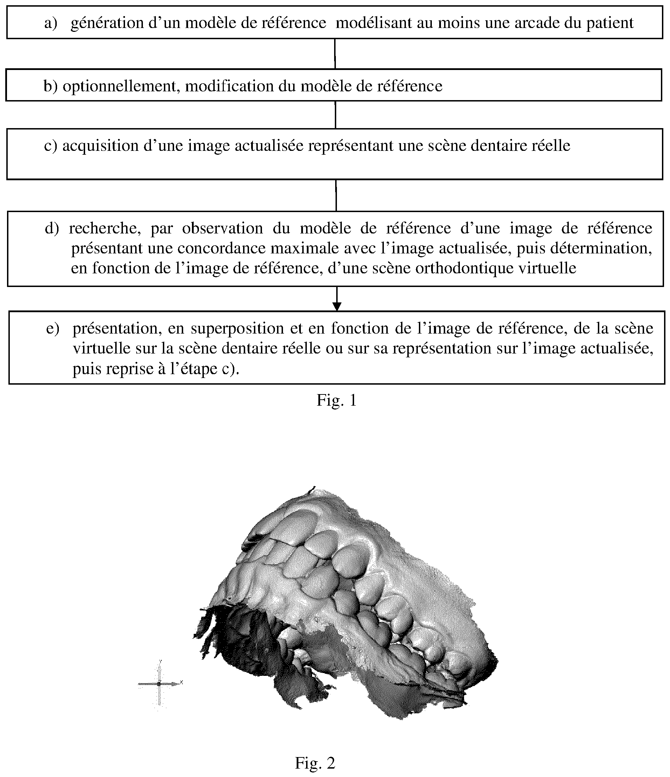

L'unité de traitement 12 peut comporter les moyens électroniques conventionnels de tout ordinateur, et en particulier une unité centrale 22, un programme et une mémoire 24. La mémoire 24 contient de préférence un modèle de référence d'au moins une partie de la scène dentaire réelle, et en particulier un modèle de l'arcade dentaire, par exemple comme représenté sur la figure 2. Le programme comporte des instructions de code permettant, lorsqu'elles sont exécutées, de mettre en oeuvre l'étape d), et en particulier d'analyser une image actualisée, de rechercher une vue du modèle de référence qui présente une concordance maximale avec l'image actualisée, c'est-à-dire une image de référence, puis de déterminer, en conséquence, une scène dentaire virtuelle.

Dans un mode de réalisation, le dispositif comporte encore une interface permettant à un opérateur de paramétrer la scène dentaire virtuelle. De préférence, l'interface est configurée pour qu'un opérateur puisse modifier un instant de simulation déterminant la scène dentaire virtuelle. Le contenu de la scène dentaire virtuelle peut en particulier être constitué par la représentation d'éléments de la scène dentaire réelle sous une apparence et/ou à une position simulées pour l'instant de simulation passé ou futur ou connue à un instant de simulation passé.

Par exemple, dans un mode de réalisation, l'écran est un écran tactile et l'opérateur peut modifier l'instant de simulation en entrant une date dans un champ de saisie ou en déplaçant un ou plusieurs doigts sur l'écran. Par exemple un déplacement vers la droite de l'écran peut conduire à avancer vers le futur et un déplacement vers la gauche de l'écran peut conduire à reculer dans le passé. La scène dentaire virtuelle présentée s'adapte en conséquence, de préférence en temps réel.

L'interface peut être également un organe mécanique, par exemple un bouton ou une mollette.

Dans un mode de réalisation, le dispositif est configuré pour que l'intervalle de temps entre l'instant actualisé et l'instant de simulation évolue continûment d'un cycle à l'autre, c'est-à-dire que la succession des cycles d'étapes A) à C) ou c) à e) simule un avancement dans le temps ou un retour dans le temps. Dans un mode de réalisation, l'écart temporel entre deux instants de simulation successifs est constant, ce qui permet de visualiser une évolution dans laquelle le temps s'écoule à vitesse constante.

L'unité de traitement détermine les conditions dans lesquelles la scène dentaire virtuelle doit être présentée pour apparaître, à l'opérateur, en superposition avec la scène dentaire réelle.

Lorsque la scène dentaire virtuelle doit être présentée sur l'image actualisée affichée sur un écran, l'opérateur observe l'écran et voit donc ce qui est observé par la caméra. De simples opérations, comme une mise à l'échelle, une correction des effets de perspective ou un recalage sur l'image actualisée, peuvent suffire pour assurer une superposition réaliste de la scène dentaire virtuelle sur l'image actualisée, par exemple en utilisant des marques caractéristiques, comme décrit ci-après.

Lorsque la scène dentaire virtuelle doit être présentée sur un écran transparent à travers lequel un opérateur observe la scène dentaire réelle, il est préférable que la caméra soit disposée de manière à capturer une image actualisée qui corresponde à la vue de la scène dentaire réelle par l'observateur. Il est également préférable que l'écran soit à une distance constante, et orienté de manière fixe par rapport à l'observateur. Avantageusement, l'unité de traitement peut ainsi déterminer facilement, à partir de l'image actualisée acquise par la caméra, la vue de la scène dentaire réelle observée par l'opérateur, ainsi que les conditions dans lesquelles la scène dentaire virtuelle doit être présentée pour apparaître, à l'opérateur, en superposition avec la scène dentaire réelle.

### Procédé

Un procédé d'analyse est mis en oeuvre au moyen d'un dispositif selon l'invention.

**A l'étape** a), à un instant de référence, on génère un modèle de référence, de préférence au moyen d'un scanner.

Le modèle de référence peut être un modèle de l'arcade du patient ou être composé d'un modèle de cette arcade et d'un modèle d'un appareil orthodontique disposé sur ledit modèle de l'arcade.

La création du modèle de référence résulte de préférence d'une prise de mesures.

De préférence, on scanne une arcade du patient avec un scanner 3D, optionnellement alors que le patient porte un appareil orthodontique, ou un moulage de cette arcade, de manière à créer un modèle de référence. De préférence, le scan est réalisé avec un scanner 3D classique.

Le modèle de référence obtenu au moyen d'un scanner représente une arcade, mais également, le cas échéant, tout objet scanné en même temps que l'arcade, par exemple un appareil orthodontique ou un article décoratif fixé sur l'arcade.

Le modèle de référence peut être alternativement un modèle générique d'arcade, choisi dans une base de modèles génériques, c'est-à-dire un modèle d'arcade applicable, de manière grossière, à plusieurs patients, ou résulter d'une déformation d'un tel modèle générique. La déformation peut en particulier être en fonction de mesures ou d'observations faites sur le patient, par exemple en fonction de photos de ses arcades dentaires. La déformation d'un modèle afin qu'il puisse correspondre à un ou plusieurs photos peut mettre en oeuvre une méthode métaheuristique, comme décrit dans WO 2016/066651 notamment.

Un modèle générique d'arcade peut être en particulier un modèle obtenu par un traitement statistique, par exemple une moyenne, sur plus de 5, plus de 10, plus de 100 ou plus de 1000 modèles historiques générés au moyen d'un scanner. Les modèles historiques sont de préférence des modèles d'arcades dentaires de patients « historiques » ayant des caractéristiques dentaires identiques ou similaires à celles du patient pour lequel on génère le modèle de référence, par exemple ayant souffert de la même pathologie et/ou ayant le même âge et/ou ayant le même sexe et/ou ayant une morphologie similaire.

Le modèle de référence, tridimensionnel, peut être observé selon un angle quelconque. Une observation du modèle, selon un angle et à une distance déterminés, est une « vue du modèle ». Les figures 2 et 3a sont des exemples de vues d'un modèle de référence.

Les vues du modèle de référence sont destinées à être comparées avec les images actualisées afin de déterminer, pour chaque image actualisée, une image de référence la plus ressemblante possible à l'image actualisée, puis constituer en conséquence une scène dentaire virtuelle apportant des informations relatives aux éléments représentés sur l'image actualisée.

L'instant de référence peut être proche de l'instant actualisé, par exemple éloigné (postérieur ou antérieur) de moins de 1 mois, 2 semaines ou 1 semaine ou 3 jours à l'instant actualisé. Des images actualisées peuvent alors être utilisées pour déformer un modèle générique jusqu'à obtenir le modèle de référence correspondant à la situation dentaire à l'instant actualisé. Par ailleurs, la scène dentaire virtuelle apporte alors des informations sur la situation dentaire propres à l'instant actualisé. Par exemple, si le modèle de référence modélise les racines des dents, la scène dentaire virtuelle peut représenter les racines des dents.

L'instant de référence peut être éloigné de l'instant actualisé, par exemple éloigné de plus de 1 mois à l'instant actualisé, 2 semaines ou 1 semaine ou 3 jours. Le modèle de référence peut alors différer sensiblement de la réalité à l'instant actualisé. Les informations fournies par la scène dentaire virtuelle peuvent alors porter sur l'évolution de la situation dentaire entre l'instant de référence et l'instant actualisé. Par exemple, elles peuvent renseigner sur un déplacement de dents ou d'un appareil orthodontique.

Par exemple, le procédé peut être utilisé pour contrôler si un appareil de contention passif maintient bien les dents en position après un traitement dentaire. Le modèle de référence peut être généré immédiatement après la pose de l'appareil et l'acquisition d'une image actualisée peut avoir lieu un mois après cette pose. L'image de référence et la scène dentaire virtuelle concerneront donc les dents dans leur position en fin de traitement et la présentation de la scène dentaire virtuelle sur la scène dentaire réelle, à l'instant actualisé, permettra ainsi au professionnel des soins dentaires de détecter les différences avec leurs positions réelles.

**A l'étape b),** dans un mode de réalisation, le modèle de référence est modifié.

La modification peut être réalisée à tout moment entre l'instant de référence et l'instant actualisé. De préférence, elle est effectuée sensiblement à l'instant de référence.

La modification du modèle de référence peut être une déformation de ce modèle.

Dans un mode de réalisation, le modèle de référence est segmenté, et en particulier des modèles de dent sont créés, comme décrit dans WO 2016/066651. Puis les modèles de dent sont déplacés ou déformés.

La modification à l'étape b) peut simuler l'effet de l'écoulement du temps, par exemple l'effet d'un traitement dentaire, entre l'instant de référence et l'instant actualisé. La modification du modèle de référence conduit ainsi à un modèle représentant les dents dans une position ou une forme estimée pour l'instant actualisé.

Le déplacement ou la déformation des modèles de dent peuvent être également déterminés ou affinés à partir d'une ou plusieurs images actualisées, comme décrit dans WO 2016/066651. De préférence, le modèle de référence initial, déterminé à l'étape a), résulte alors de mesures au moyen d'un scan d'au moins une arcade ou d'au moins un moulage d'une arcade.

Le procédé peut être ainsi utilisé pour vérifier si le déroulement d'un traitement dentaire est conforme aux prévisions, le modèle de référence initial étant modifié pour correspondre à la situation anticipée à l'instant actualisé. En orthodontie, les évolutions sensibles peuvent prendre quelques jours. De préférence, dans ce mode de réalisation, l'intervalle de temps entre l'instant actualisé et l'instant de référence est supérieur à 3 jours, voire supérieur à 1 semaine, supérieur à 2 semaines, supérieur à 4 semaines, supérieur à 8 semaines, supérieur à 12 semaines, voire supérieur à 16 semaines.

L'image de référence, résultant d'une observation du modèle de référence modifié fait alors apparaître les dents dans leurs positions ou formes anticipées à l'instant actualisé. Elle constitue ou peut être intégrée dans une scène dentaire virtuelle dont la présentation, à l'étape e), permet au professionnel des soins dentaires de détecter les différences entre les positions ou formes réelles des dents et les positions ou formes anticipées pour l'instant actualisé.

La modification du modèle de référence initial peut consister en un ajout d'un autre modèle et/ou dans la modification de cet autre modèle. Par exemple, un modèle d'un appareil orthodontique posé sur l'arcade peut être ajouté à un modèle de référence qui ne représenterait initialement que l'arcade. La modification du modèle de référence peut alors inclure une modification de cet appareil orthodontique pour qu'il apparaisse dans sa position ou forme anticipée pour l'instant actualisé. Sa présentation, à l'étape e), permet alors au professionnel des soins dentaires de détecter les différences entre la position ou forme réelle de l'appareil orthodontique et la position ou forme anticipées pour l'instant actualisé, et notamment de détecter tout décollement d'une attache.

**A l'étape c**), à l'instant actualisé, on acquiert avec la caméra une image actualisée représentant une scène dentaire réelle. Sur les figures, l'image actualisée est représentée en trait continu sur l'écran 18.

Un dispositif selon l'invention dans lequel la caméra est fixée sur la monture de lunettes ou est intégrée dans un miroir permet d'acquérir une image actualisée qui représente avec une grande précision la scène dentaire réelle observée par l'opérateur à travers les verres de ces lunettes ou sur ce miroir.

Un dispositif selon l'invention dans lequel la caméra est intégrée dans un téléphone portable, un ordinateur portable, une tablette ou un appareil photo permet d'acquérir une image actualisée qui représente exactement la scène dentaire réelle observée par l'opérateur sur l'écran du téléphone portable, de l'ordinateur, de la tablette, ou de l'appareil photo respectivement.

De préférence, l'image actualisée est en couleurs, de préférence en couleurs réelles.

**A l'étape d),** on recherche une image de référence qui présente une concordance maximale avec l'image actualisée.

A l'étape d), toutes les méthodes connues pour réaliser une concordance maximale sont envisagées.

L'obtention d'une concordance maximale peut notamment résulter d'une optimisation de manière à minimiser ladite distance (« *bestfit »).*

De préférence, l'image de référence est recherchée en observant le modèle de référence selon différents angles d'observation et différentes distances, jusqu'à obtenir une vue du modèle de référence qui corresponde sensiblement à l'image actualisée, c'est-à-dire qui puisse lui être sensiblement superposée. Pour chaque image actualisée, on obtient ainsi une image de référence présentant une concordance maximale avec l'image actualisée. Cette recherche est de préférence effectuée au moyen d'une méthode métaheuristique, de préférence évolutionniste, de préférence par recuit simulé.

Les méthodes métaheuristiques sont des méthodes d'optimisation connues. Elles sont de préférence choisies dans le groupe formé par
- les algorithmes évolutionnistes, de préférence choisie parmi: les stratégies d'évolution, les algorithmes génétiques, les algorithmes à évolution différentielle, les algorithmes à estimation de distribution, les systèmes immunitaires artificiels, la recomposition de chemin Shuffled Complex Evolution, le recuit simulé, les algorithmes de colonies de fourmis, les algorithmes d'optimisation par essaims particulaires, la recherche avec tabous, et la méthode GRASP ;
- l'algorithme du kangourou,
- la méthode de Fletcher et Powell,
- la méthode du bruitage,
- la tunnelisation stochastique,
- l'escalade de collines à recommencements aléatoires,
- la méthode de l'entropie croisée, et
- les méthodes hybrides entre les méthodes métaheuristiques citées ci-dessus.

L'exploration du modèle de référence pour rechercher l'image de référence peut comprendre une ou plusieurs des caractéristiques des étapes c), d) et e) de WO 2016 066651, dans la mesure où elles concernent une telle exploration.

De préférence, on traite l'image actualisée pour réaliser une carte actualisée représentant, au moins partiellement, une information discriminante, et ladite recherche comporte les étapes suivantes :
i) acquisition d'une vue du modèle de référence ;
ii) traitement de la vue pour réaliser au moins une carte de référence représentant, au moins partiellement, l'information discriminante ;
iii) comparaison des cartes actualisée et de référence de manière à déterminer une distance entre lesdites cartes actualisée et de référence et, si la distance est supérieure à un seuil, modification de la vue, puis reprise à l'étape ii).

Les cartes actualisée et de référence représentent la même information discriminante. L'information discriminante est une information caractéristique qui peut être extraite d'une image *("image feature"),* classiquement par un traitement informatique de cette image. Elle est de préférence choisie dans le groupe constitué par une information de contour, une information de couleur, une information de densité, une information de distance, une information de brillance, une information de saturation, une information sur les reflets et des combinaisons de ces informations.

L'homme de l'art sait comment traiter une image pour faire apparaître l'information discriminante, et donc créer la carte correspondante.

Par exemple, la figure 4b est une carte actualisée relative au contour des dents obtenue à partir de l'image actualisée de la figure 4a. La figure 3b est une carte de référence relative au contour des dents obtenue à partir de la vue de la figure 3a.

La modification de la vue est de préférence guidée par des règles heuristiques, par exemple en favorisant les modifications qui, d'après une analyse des précédentes distances, apparaissent les plus favorables pour la réduire.

L'étape iii) aboutit à une image de référence sensiblement superposable à l'image actualisée.

L'obtention d'une concordance maximale entre l'image de référence et l'image actualisée peut également résulter de la superposition de repères identifiés sur ces deux images, par exemple la superposition de repères fixes comme l'extrémité d'une dent ou un point de contact entre deux dents.

La scène dentaire virtuelle comporte de préférence, voire est constituée de l'image de référence. L'image de référence est un exemple particulier d'une scène dentaire virtuelle.

Sur la figure 8, les éléments de la scène dentaire virtuelle sont représentés en trait interrompu.

Dans un mode de réalisation préféré, une scène dentaire virtuelle comporte une représentation, de préférence réaliste, d'un ou plusieurs éléments de la scène dentaire réelle correspondante. En particulier, de préférence, elle comporte une représentation de l'arcade, et une représentation d'un appareil orthodontique porté par les dents de l'arcade.

Comme représenté sur la figure 8, la scène dentaire virtuelle peut par exemple représenter les contours 26 des dents.

La scène dentaire virtuelle peut également comporter la représentation d'un ou plusieurs éléments physiques de la scène dentaire réelle non modélisés par le modèle de référence. Pour identifier de tels éléments physiques, l'image actualisée est analysée, par exemple au moyen d'algorithmes d'intelligence artificielle.

La représentation d'un élément physique peut être réaliste, par exemple être constituée des contours de l'élément physique considéré, ou symbolique. La représentation des éléments physiques de la scène dentaire virtuelle peut être bidimensionnelle et/ou tridimensionnelle. La présentation de la scène dentaire virtuelle peut être en particulier un hologramme.

La scène dentaire virtuelle peut également comporter, en complément ou alternativement à la représentation d'éléments physiques, par exemple de l'image de référence, un ou plusieurs indicateurs 34.

Un indicateur est un élément d'une scène dentaire virtuelle qui ne représente pas, même symboliquement, un élément physique de la scène dentaire réelle.

Un indicateur peut être en particulier un texte ou un symbole (un point, un trait, une flèche, un aplat de couleur...), par exemple un symbole illustrant des différences entre l'image actualisée et l'image de référence.

La nature d'un indicateur peut être déterminée en fonction de l'image de référence. Les indicateurs peuvent être en particulier associés au modèle de référence ou à une région du modèle de référence. Ils sont par exemple stockés dans une base de données dans la mémoire 24 de l'unité de traitement.

Par exemple, des informations complémentaires peuvent être associées à différentes régions du modèle de référence, par exemple pour indiquer des zones de collage pour un appareil orthodontique. Des informations complémentaires peuvent être également associées au modèle de référence dans son ensemble, par exemple pour préciser les circonstances de sa réalisation ou des informations sur le patient ou sur l'appareil orthodontique. Les indicateurs permettent de présenter ces informations complémentaires. La nature d'un indicateur peut être déterminée en fonction des différences entre l'image actualisée et l'image de référence.

Par exemple, des indicateurs peuvent être ajoutés pour mettre en évidence un détachement d'un appareil orthodontique.

La nature et/ou l'emplacement et/ou l'apparence d'un indicateur peut être déterminée en fonction du contexte, et en particulier en fonction de la distance à un autre élément de la scène dentaire virtuelle et/ou en fonction d'un objectif poursuivi. Par exemple, il apparaîtra avec une apparence fonction de la distance à une dent particulière ou fonction du risque pour le patient.

Le contexte est défini par les conditions dans lesquelles le procédé est utilisé.

Par exemple, l'opérateur peut avoir pour objectif de vérifier le positionnement de l'appareil orthodontique porté par le patient. Les indicateurs qui ne sont pas liés à cette vérification, par exemple l'indication des zones de carie, ne sont alors pas nécessaires.

Dans un mode de réalisation, avant l'étape d), l'opérateur saisit des informations complémentaires dans la base de données, en fonction de l'utilisation du procédé visée. Les indicateurs correspondants peuvent avantageusement être ajoutés à la scène dentaire virtuelle.

Les éléments qui constituent la scène dentaire virtuelle sont positionnés dans cette scène en fonction des éléments de la scène dentaire réelle, et donc en l'occurrence, en fonction de l'image de référence. En particulier, les représentations d'éléments physiques réels sont de préférence positionnées dans la scène dentaire virtuelle de manière qu'à l'étape e), ils se superposent, de manière précise, avec ces éléments physiques réels ou avec leurs représentations sur l'image actualisée.

De même, un indicateur apportant une information spécifique à un élément physique, par exemple spécifique à une dent, est de préférence positionné de manière qu'à l'étape e) il apparaisse à proximité immédiate ou superposé à cet élément physique, par exemple à cette dent.

Des indicateurs peuvent être disposés de manière à être affichés, à l'étape suivante, aux endroits où des différences ont été identifiées entre l'image de référence et l'image actualisée. Par exemple, on peut afficher une marque rouge aux emplacements où un appareil orthodontique s'est décollé des dents.

Dans un mode de réalisation, le contexte est évolutif et la nature et/ou l'emplacement, dans la scène dentaire virtuelle, des indicateurs sont adaptés en conséquence. En particulier, la nature et/ou l'emplacement des indicateurs peuvent être déterminés en fonction de la nature et/ou de l'emplacement d'indicateurs affichés dans des scènes dentaires virtuelles antérieures, c'est-à-dire déterminées lors de cycles c)-e) (ou A) à C)) précédents, et/ou en fonction de l'intervalle temporel avec lesdites scènes dentaires virtuelles antérieures, et/ou en fonction de différences entre les images actualisées de cycles c)-e) (ou A) à C)) successifs.

Dans un mode de réalisation, l'apparence d'un élément de la scène dentaire virtuelle évolue en fonction du contexte.

Par exemple, on identifie un élément de la scène dentaire virtuelle qui ne respecte pas un critère d'évaluation, par exemple parce qu'il dépasse un seuil de risque, puis on attire l'attention vers cet élément dans la scène dentaire virtuelle. Par exemple, la couleur de l'élément peut être modifiée si l'analyse du contexte montre que la situation devient dangereuse, par exemple parce que l'appareil orthodontique conduirait à une correction excessive du positionnement des dents.

Dans un mode de réalisation, la scène dentaire virtuelle ne comporte pas l'image de référence. L'image de référence peut être utilisée pour déterminer la nature et/ou l'emplacement des éléments dans la scène dentaire virtuelle. Mais elle n'est pas présentée à l'observateur à l'étape e) (ou C)).

Dans un mode de réalisation, la scène dentaire virtuelle est déterminée en fonction d'un instant de simulation passé ou futur. En particulier, elle peut comporter une représentation, de préférence réaliste, d'un ou plusieurs éléments de la scène dentaire réelle correspondante dans une configuration passée ou future.

Dans un mode de réalisation, elle comporte une représentation de l'arcade et/ou une représentation d'un appareil orthodontique porté par les dents de l'arcade telle que mesurée ou simulée à un instant de simulation passé.

Dans un mode de réalisation, elle comporte une représentation de l'arcade et/ou une représentation d'un appareil orthodontique porté par les dents de l'arcade telle qu'anticipée pour un instant de simulation futur.

La simulation à un instant de simulation passé ou futur peut être réalisée par tout logiciel de simulation ou par un opérateur, par exemple par un professionnel des soins dentaires.

La scène dentaire virtuelle peut par exemple représenter les contours des dents, le positionnement d'un appareil orthodontique ou la couleur des dents à l'instant de simulation. La présentation de la scène dentaire virtuelle sur la scène dentaire réelle, de préférence en transparence sur la scène dentaire réelle, permet ainsi de facilement détecter l'évolution entre l'instant actualisé et l'instant de simulation.

Lorsqu'un indicateur représente une partie non visible d'un élément physique de la scène dentaire réelle, par exemple une racine d'une dent, l'information relative à cet indicateur est de préférence incluse dans le modèle de référence. Par exemple, le modèle de référence peut être un modèle de l'arcade dentaire dans lequel les couronnes des dents, mais aussi les racines des dents sont modélisées, par exemple au moyen d'un scanner à faisceau conique *(« Cone beam* »). Le procédé selon l'invention permet ainsi à l'opérateur de visualiser des parties de l'arcade dentaire du patient qui ne sont pas visibles sur la scène dentaire réelle. Avantageusement, ces parties non visibles sont représentées dans leur position relative réelle par rapport aux éléments physiques de la scène dentaire réelle. Par exemple, l'opérateur voit les racines et les nerfs des dents qu'il observe dans la bouche du patient.

**A l'étape** e), on présente, en fonction de l'image de référence, la scène dentaire virtuelle en superposition sur la scène dentaire réelle ou sur la représentation de la scène dentaire réelle dans l'image actualisée.

La scène dentaire virtuelle peut être directement projetée sur la scène dentaire réelle.

La scène dentaire virtuelle est affichée sur un écran.

Lorsque l'écran est transparent, en particulier dans le mode de réalisation dans lequel il est intégré dans des lunettes, l'opérateur peut ainsi observer la scène dentaire virtuelle sur l'écran et la scène dentaire réelle à travers l'écran, en transparence derrière la scène dentaire virtuelle.

Lorsque l'écran est opaque, en particulier dans le mode de réalisation dans lequel l'écran est l'écran d'un téléphone portable, d'un ordinateur portable, d'un casque de réalité virtuelle ou d'une tablette, l'opérateur peut ainsi simultanément observer sur l'écran l'image actualisée et la scène dentaire virtuelle.

La scène dentaire virtuelle peut être suffisamment transparente pour laisser apparaître la scène dentaire réelle ou l'image actualisée sous elle.

Alternativement, la scène dentaire virtuelle peut être opaque, de manière à ne pas laisser apparaître la scène dentaire réelle ou l'image actualisée qui s'étend derrière ou sous elle. L'opacité de la scène dentaire virtuelle est particulièrement avantageuse pour simuler une situation dentaire passée ou future. Lorsque la scène dentaire virtuelle est présentée sur l'image actualisée, elle peut être ajoutée au-dessus de l'image actualisée ou, de manière équivalente, l'image actualisée peut être remplacée par une image retraitée informatiquement pour faire apparaître la scène dentaire virtuelle.

Par « présentation de la scène dentaire virtuelle en superposition sur la représentation de la scène dentaire réelle dans l'image actualisée », il faut donc inclure toute présentation faisant apparaître la scène dentaire virtuelle sur la représentation de la scène dentaire réelle, y compris l'affichage d'une image retraitée.

Lorsque l'écran est la glace d'un miroir, l'opérateur peut simultanément observer sur l'écran son reflet et la scène dentaire virtuelle, superposée à son reflet. En particulier, le patient peut visualiser l'effet d'un traitement dentaire, thérapeutique ou non.

La scène dentaire virtuelle est présentée en fonction de l'image de référence et donc, indirectement, en fonction de l'image actualisée. En particulier, elle est positionnée, ou « cadrée », par rapport à la scène dentaire réelle en fonction de l'image de référence.

La scène dentaire virtuelle est présentée en superposition, ou « en registre », avec la scène dentaire réelle ou avec sa représentation sur l'image actualisée, ce qui facilite la comparaison. Autrement dit, les éléments de la scène dentaire virtuelle qui correspondent à des éléments de la scène dentaire réelle sont sensiblement exactement superposés à ces derniers ou aux représentations de ces derniers. Notamment lorsque la scène dentaire virtuelle est présentée en transparence, les différences entre la scène dentaire réelle ou sa représentation d'une part et la scène dentaire virtuelle d'autre part apparaissent ainsi clairement à l'opérateur. Les indicateurs lui présentent par ailleurs des informations facilitant l'analyse de la situation.

De préférence, pour présenter la scène dentaire virtuelle en registre sur la scène dentaire réelle ou sur sa représentation sur l'image actualisée, on identifie sur l'image actualisée des marques caractéristiques dont la position par rapport à la scène dentaire virtuelle est connue. Par exemple, on identifie des extrémités de dent ou des formes caractéristiques de dents représentées à la fois sur l'image actualisée et sur la scène dentaire virtuelle. L'image de référence à partir de laquelle la scène dentaire virtuelle a été déterminée peut en particulier être utilisée pour identifier la position des marques caractéristiques dans la scène virtuelle. A l'étape e) (ou C)), on superpose ensuite les représentations de ces marques caractéristiques sur l'image actualisée et dans la scène dentaire virtuelle, ce qui permet de positionner précisément la scène dentaire virtuelle par rapport aux marques caractéristiques représentées sur l'image actualisée.

Le procédé reprend ensuite à l'étape c) (ou A)), moins de 5 secondes, de préférence moins de 3 secondes, de préférence moins de 1 s, moins de 0,5 s, moins de 0,2 s, de préférence moins de 0,1 s après l'étape e) (ou C)). De préférence, le cycle des étapes c) à e) (ou A) à C)) est effectué en temps réel, sans interruption.

De manière générale, l'invention peut être utilisée pour évaluer la situation dentaire du patient, notamment pour contrôler le déroulement d'un traitement dentaire.

Bien entendu, l'invention n'est pas limitée aux modes de réalisation décrits ci-dessus et représentés.

En particulier, le patient n'est pas limité à un être humain. Un procédé peut être utilisé pour un autre animal.

Dans un procédé comportant des étapes a) à e), la recherche d'une image de référence n'est pas indispensable à la mise en oeuvre du dispositif de l'invention. Si une image de référence n'est pas disponible, les éléments qui constituent la scène dentaire virtuelle peuvent être positionnés sur la scène dentaire réelle ou sa représentation, en fonction de l'image actualisée.

Une scène dentaire virtuelle peut être déterminée en fonction de plusieurs images de référence obtenues à partir de plusieurs images actualisées respectives acquises simultanément ou à des instants actualisés différents, par exemple successifs.

## Revendications

1. Dispositif pour la mise en oeuvre d'un procédé d'analyse d'une situation dentaire réelle d'un patient, le procédé comportant une série de cycles d'étapes A) à C), un cycle d'étapes A) à C) comportant les étapes successives suivantes :
A) à un instant actualisé, acquisition d'une image actualisée représentant une scène dentaire réelle comportant un appareil orthodontique porté par le patient telle qu'observée par un opérateur ;
B) détermination d'une scène dentaire virtuelle en fonction de la représentation de la scène dentaire réelle sur l'image actualisée, la scène dentaire virtuelle comportant une représentation de l'appareil orthodontique porté par le patient, et/ou des informations sur un emplacement cible pour l'appareil orthodontique ; et
identification d'un élément de la scène dentaire virtuelle qui ne respecte pas un critère d'évaluation, et mise en évidence de cet élément dans la scène dentaire virtuelle et/ou ajout à la scène dentaire virtuelle d'un message relatif audit élément, le critère d'évaluation étant relatif au positionnement de l'appareil orthodontique porté par le patient et/ou à l'état de l'appareil orthodontique porté par le patient, ; puis
C) présentation, sur un écran, de la scène dentaire virtuelle en transparence et en superposition sur la scène dentaire réelle, ou
affichage de l'image actualisée sur un écran et présentation de la scène dentaire virtuelle en superposition avec la représentation de la scène dentaire réelle sur l'image actualisée affichée sur l'écran, en transparence ou non sur ladite représentation ;
une étape A) étant reprise moins de 5 secondes après l'étape C),
le dispositif comportant :
- une caméra (16) configurée pour mettre en oeuvre l'étape A) ;
- une unité de traitement (12) configurée pour mettre en oeuvre l'étape B) ;
- un écran (18) pour mettre en oeuvre l'étape C) ;
la caméra (16), l'unité de traitement (12) et l'écran (18) étant configurés pour communiquer entre eux pour mettre en oeuvre lesdites étapes.

2. Dispositif selon la revendication immédiatement précédente, dans lequel, la scène dentaire virtuelle est relative à la scène dentaire réelle telle que simulée à un instant de simulation, l'instant de simulation est écarté de l'instant actualisé de plus de 3 jours.

3. Dispositif selon l'une quelconque des revendications précédentes, dans lequel la caméra (16) et/ou l'écran (18) sont intégrés dans un téléphone portable, un ordinateur portable, un casque de réalité virtuelle, une tablette, un appareil photo ou un miroir (17), la glace (19) du miroir, de préférence sans tain, constituant ledit écran, ou dans lequel l'écran et la caméra sont indépendants l'un de l'autre, c'est-à-dire ne sont pas rigidement solidaire l'un de l'autre.

4. Dispositif selon l'une quelconque des revendications précédentes, dans lequel, à l'étape B), une couleur et/ou une forme et/ou une dimension et/ou une position d'une partie visible ou invisible de la bouche, de préférence d'une mâchoire, d'une couronne dentaire et/ou d'une racine dentaire et/ou de la gencive du patient, est/sont déterminée(s) de manière que la scène dentaire virtuelle reproduise ladite couleur et/ou forme et/ou position telle(s) que simulée(s) à un instant de simulation.

5. Dispositif selon l'une quelconque des revendications précédentes, dans lequel, à l'étape B), la scène dentaire virtuelle est déterminée en fonction d'une valeur d'au moins un paramètre de traitement modifiable par un opérateur.

6. Dispositif selon la revendication immédiatement précédente, dans lequel le paramètre de traitement est relatif à un port d'un appareil orthodontique par le patient et/ou au respect d'une consigne de traitement par le patient.

7. Dispositif selon l'une quelconque des revendications précédentes, dans lequel le procédé d'analyse comporte les étapes successives suivantes :
a) à un instant de référence, génération d'un modèle de référence modélisant numériquement au moins une arcade du patient;
b) modification du modèle de référence ;
c) à l'instant actualisé, acquisition de ladite image actualisée, suivant l'étape A) ;
d) recherche, par observation du modèle de référence modifié, d'une image de référence présentant une concordance maximale avec l'image actualisée, puis détermination, en fonction de l'image de référence, de la scène dentaire virtuelle, suivant l'étape B) ;
e) présentation de la scène dentaire virtuelle suivant l'étape C),
le dispositif comportant des moyens pour générer et modifier le modèle de référence suivant les étapes a) et b), ces moyens comportant de préférence un scanner et un ordinateur,
la caméra étant configurée pour mettre en oeuvre l'étape c), l'unité de traitement pour mettre en oeuvre l'étape d) et l'écran pour mettre en oeuvre l'étape e).

8. Dispositif selon la revendication immédiatement précédente, dans lequel, à l'étape b), le modèle de référence est modifié pour simuler l'effet de l'écoulement du temps entre l'instant de référence et un instant de simulation.

9. Dispositif selon l'une quelconque des revendications précédentes configuré pour simuler :
le bon positionnement d'un appareil orthodontique fixé sur les dents.

10. Dispositif selon l'une quelconque des revendications précédentes, dans lequel le message est une consigne à suivre par le patient et/ou par un professionnel des soins dentaires en charge du patient.

11. Dispositif selon l'une quelconque des revendications précédentes, dans lequel, la scène dentaire virtuelle est directement projetée sur la scène dentaire réelle.

12. Dispositif selon l'une quelconque des revendications précédentes, dans lequel, la caméra (16) et l'écran (18) étant intégrés dans un téléphone portable ou un ordinateur portable ou une tablette.

## Patentansprüche

1. Vorrichtung für die Durchführung eines Verfahrens für eine Analyse einer tatsächlichen Dentalsituation eines Patienten, wobei das Verfahren eine Reihe von Zyklen der Schritte A) bis C) umfasst, wobei ein Zyklus der Schritte A) bis C) die folgenden aufeinanderfolgenden Schritte umfasst:
A) zu einem aktualisierten Zeitpunkt, Erfassen eines aktualisierten Bilds, das eine tatsächliche Dentalszene darstellt, die eine durch den Patienten getragene kieferorthopädische Apparatur umfasst, wie sie durch einen Bediener beobachtet wird;
B) Bestimmen einer virtuellen Dentalszene in Abhängigkeit von der Darstellung der tatsächlichen Dentalszene auf dem aktualisierten Bild, wobei die virtuelle Dentalszene eine Darstellung der durch den Patienten getragenen kieferorthopädischen Apparatur und/oder Informationen zu einer Zielposition für die kieferorthopädische Apparatur umfasst; und Identifizieren eines Elements der virtuellen Dentalszene, das ein Bewertungskriterium nicht berücksichtigt, und
Nachweisen dieses Elements in der virtuellen Dentalszene und/oder Hinzufügen einer Nachricht relativ zu dem Element zu der virtuellen Dentalszene, wobei das Bewertungskriterium relativ zu der Positionierung der durch den Patienten getragenen kieferorthopädischen Apparatur und/oder zu dem Zustand der durch den Patienten getragenen kieferorthopädischen Apparatur; dann
C) transparentes und überlagerndes Darstellen der virtuellen Dentalszene über der tatsächlichen Dentalszene auf einem Bildschirm, oder
Anzeigen des aktualisierten Bilds auf einem Bildschirm und überlagerndes Darstellen der virtuellen Dentalszene mit der Darstellung der tatsächlichen Dentalszene auf dem aktualisierten, auf dem Bildschirm angezeigten, Bild, wobei die Darstellung transparent oder nicht ist;
wobei Schritt A) weniger als 5 Sekunden nach Schritt C) wieder aufgenommen wird, wobei die Vorrichtung umfasst:
- eine Kamera (16), die konfiguriert ist, um Schritt A) durchzuführen;
- eine Verarbeitungseinheit (12), die konfiguriert ist, um Schritt B) durchzuführen;
- einen Bildschirm (18) zum Durchführen von Schritt C);
wobei die Kamera (16), die Verarbeitungseinheit (12) und der Bildschirm (18) zum Kommunizieren untereinander zum Durchführen der Schritte konfiguriert sind.

2. Vorrichtung nach dem unmittelbar vorstehenden Anspruch, wobei die virtuelle Dentalszene relativ zu der tatsächlichen Dentalszene ist, wie sie zu einem Simulationszeitpunkt simuliert wird, wobei der Simulationszeitpunkt von dem aktualisierten Zeitpunkt um mehr als 3 Tage getrennt ist.

3. Vorrichtung nach einem der vorstehenden Ansprüche, wobei die Kamera (16) und/oder der Bildschirm (18) in ein Mobiltelefon, einen Laptop, ein Virtual-Reality-Headset, ein Tablet, eine Fotokamera oder einen Spiegel (17) integriert ist, wobei das Glas (19) des Spiegels, vorzugsweise ein Spionspiegel, den Bildschirm bildet, oder wobei der Bildschirm und die Kamera unabhängig voneinander sind, das heißt, nicht fest miteinander verbunden sind.

4. Vorrichtung nach einem der vorstehenden Ansprüche, wobei in Schritt B) eine Farbe und/oder eine Form und/oder eine Abmessung und/oder eine Position eines sichtbaren oder unsichtbaren Teils des Mundes, vorzugsweise eines Kiefers, einer Zahnkrone und/oder einer Zahnwurzel und/oder des Zahnfleischs des Patienten, bestimmt wird/werden, sodass die virtuelle Dentalszene die Farbe und/oder die Form und/oder die Position wie zu einem Simulationszeitpunkt simuliert wiedergibt.

5. Vorrichtung nach einem der vorstehenden Ansprüche, wobei in Schritt B) die virtuelle Dentalszene in Abhängigkeit von einem Wert mindestens eines durch einen Bediener veränderbaren Verarbeitungsparameters bestimmt wird.

6. Vorrichtung nach dem unmittelbar vorstehenden Anspruch, wobei der Behandlungsparameter relativ zu dem Tragen einer kieferorthopädischen Apparatur durch den Patienten und/oder in Bezug auf eine Behandlungstreue durch den Patienten ist.

7. Vorrichtung nach einem der vorstehenden Ansprüche, wobei das Analyseverfahren die folgenden aufeinanderfolgenden Schritte umfasst:
a) zu einem Referenzzeitpunkt, Erzeugen eines Referenzmodells, das mindestens einen Zahnbogen des Patienten digital modelliert;
b) Modifizieren des Referenzmodells;
c) zu dem aktualisierten Zeitpunkt, Erfassen des aktualisierten Bilds gemäß Schritt A);
d) Suchen, durch Beobachtung des modifizierten Referenzmodells, nach einem Referenzbild, das eine maximale Übereinstimmung mit dem aktualisierten Bild aufweist, dann Bestimmen,
in Abhängigkeit von dem Referenzbild, der virtuellen Dentalszene, gemäß Schritt B);
e) Darstellen der virtuellen Dentalszene gemäß Schritt C),
wobei die Vorrichtung Mittel zum Erzeugen und Modifizieren des Referenzmodells gemäß den Schritten a) und b) umfasst, wobei diese Mittel vorzugsweise einen Scanner und einen Computer umfassen,
wobei die Kamera zum Durchführen von Schritt c) konfiguriert ist, die Verarbeitungseinheit zum Durchführen von Schritt d) und der Bildschirm zum Durchführen von Schritt e).

8. Vorrichtung nach dem unmittelbar vorstehenden Anspruch, wobei in Schritt b) das Referenzmodell zum Simulieren der Zeitablaufwirkung zwischen dem Referenzzeitpunkt und einem Simulationszeitpunkt modifiziert wird.

9. Vorrichtung nach einem der vorstehenden Ansprüche, die konfiguriert ist zum Simulieren von:
der korrekten Positionierung einer auf den Zähnen befestigten kieferorthopädischen Apparatur.

10. Vorrichtung nach einem der vorstehenden Ansprüche, wobei die Nachricht eine Anweisung ist, die durch den Patienten und/oder durch einen für den Patienten zuständigen Zahnarzt zu befolgen ist.

11. Vorrichtung nach einem der vorstehenden Ansprüche, wobei die virtuelle Dentalszene direkt auf die tatsächliche Dentalszene projiziert wird.

12. Vorrichtung nach einem der vorstehenden Ansprüche, wobei die Kamera (16) und der Bildschirm (18) in ein Mobiltelefon oder einen Laptop oder ein Tablet integriert sind.

## Claims

1. Device for implementing a method for analyzing a patient's real dental situation, the method comprising a series of cycles of steps A) to C), a cycle of steps A) to C) comprising the following successive steps:
A) at an up-to-date time, acquiring an up-to-date image representing a real dental scene comprising an orthodontic appliance worn by the patient as observed by an operator;
B) determining a virtual dental scene on the basis of the representation of the real dental scene on the up-to-date image, the virtual dental scene comprising a representation of the orthodontic appliance worn by the patient, and/or information on a target location for the orthodontic appliance; and identifying an element of the virtual dental scene which does not comply with an evaluation criterion, and
highlighting this element in the virtual dental scene and/or adding a message relating to said element to the virtual dental scene, the evaluation criterion relating to the positioning of the orthodontic appliance worn by the patient and/or the state of the orthodontic appliance worn by the patient; then
C) presenting, on a screen, the virtual dental scene transparently and superimposed over the real dental scene, or
displaying the up-to-date image on a screen and presenting the virtual dental scene superimposed over the representation of the real dental scene on the up-to-date image displayed on the screen, transparently or not over said representation;
step A) being repeated less than 5 seconds after step C), the device comprising:
- a camera (16) configured to implement step A);
- a processing unit (12) configured to implement step B);
- a screen (18) for implementing step C);
the camera (16), processing unit (12) and screen (18) being configured to communicate with one another in order to implement said steps.

2. Device according to the immediately preceding claim, wherein the virtual dental scene relates to the real dental scene as simulated at a simulation time, and the simulation time is more than 3 days away from the up-to-date time.

3. Device according to any one of the preceding claims, wherein the camera (16) and/or the screen (18) are integrated into a cell phone, a laptop, a virtual reality headset, a tablet, a camera or a mirror (17), the glass (19) of the mirror, which is preferably one-way, constituting said screen, or wherein the screen and the camera are independent of one another, i.e. are not rigidly connected to one another.

4. Device according to any one of the preceding claims, wherein, in step B), a color and/or a shape and/or a dimension and/or a position of a visible or invisible part of the mouth, preferably of a jaw, a dental crown and/or a dental root and/or the gum of the patient, is/are determined in such a way that the virtual dental scene reproduces said color and/or shape and/or position as simulated at a simulation time.

5. Device according to any one of the preceding claims, wherein, in step B), the virtual dental scene is determined on the basis of a value of at least one treatment parameter that can be modified by an operator.

6. Device according to the immediately preceding claim, wherein the treatment parameter relates to the patient wearing an orthodontic appliance and/or to the patient's compliance with a treatment instruction.

7. Device according to any one of the preceding claims, wherein the analysis method comprises the following successive steps:
a) at a reference time, generating a reference model that digitally models at least one of the patient's arches;
b) modifying the reference model;
c) at the up-to-date time, acquiring said up-to-date image according to step A);
d) searching, by observing the modified reference model, for a reference image that matches the up-to-date image as closely as possible, then determining,
on the basis of the reference image, the virtual dental scene according to step B);
e) presenting the virtual dental scene according to step C),
the device comprising means for generating and modifying the reference model according to steps a) and b), these means preferably comprising a scanner and a computer,
the camera being configured to implement step c), the processing unit to implement step d) and the screen to implement step e).

8. Device according to the immediately preceding claim, wherein, in step b), the reference model is modified to simulate the effect of the passage of time between the reference time and a simulation time.

9. Device according to any one of the preceding claims, configured to simulate:
the correct positioning of an orthodontic appliance attached to the teeth.

10. Device according to any one of the preceding claims, wherein the message is an instruction to be followed by the patient and/or by a dental care professional in charge of the patient.

11. Device according to any one of the preceding claims, wherein the virtual dental scene is projected directly onto the real dental scene.

12. Device according to any one of the preceding claims, wherein the camera (16) and the screen (18) are integrated into a cell phone or a laptop or a tablet.
